# EUROPEAN PATENT APPLICATION

(11) **EP 4 537 878 A1**
(43) Date of publication of application: **16.04.2025**
(21) Application number: 23202305.1
(22) Date of filing: 09.10.2023
(51) Int. Cl.: A61M 16/00, A61B 5/08, G16H 10/00, G16H 20/00

(54) **COMPUTER PROGRAM PRODUCT, RESPIRATORY SUPPORT DEVICE, METHOD, AND DATA PROCESSING SYSTEM**

(71) Applicant: Koninklijke Philips N.V., 5656 AG Eindhoven (NL)
(72) Inventor: MENA BENITO, Maria Estrella, Eindhoven (NL); WEIJSEN, Tim Elisabeth Joseph, Eindhoven (NL); TSONEVA, Tsvetomira Kirova, 5656AG Eindhoven (NL); WESTERINK, Joanne Henriëtte Desirée Monique, Eindhoven (NL); JUAN, Elsa, Eindhoven (NL); KAHLERT, Joachim Johannes, Eindhoven (NL)
(74) Representative: Philips Intellectual Property & Standards

(57) **Abstract**

There is provided a computer program product, comprising instructions which, when executed by a processing unit (110), cause the processing unit to carry out a method (400) for controlling a respiratory support device (100) to provide a pressurized airflow (102) to a subject (104). The method comprises the steps of receiving a respiratory signal (121) representative of respiratory information of the subject, determining at least one of a hypoxia and a hypercapnia based on the respiratory signal, and providing a control signal (140) to the respiratory support device in response to determining the at least one of a hypoxia and a hypercapnia. Further, there is provided a respiratory support device for providing a pressurized airflow to a subject.

## Description

### FIELD OF THE INVENTION

The invention relates to a computer program product, comprising instructions which, when executed by a processing unit, cause the processing unit to carry out a method for controlling a respiratory support device to provide a pressurized airflow to a subject. Further, the invention relates to a respiratory support device for providing a pressurized airflow to a subject. Further, the invention relates to a method for providing a further desired property for a further respiratory support device to provide a further pressurized airflow with the further desired property to a further subject, and to a data-processing system configured to perform this method.

### BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

OSA is a respiratory condition in which pharyngeal airway collapse causes brief, episodic reductions in intrathoracic airflow while sleeping. This results in cyclical oxygen desaturation, frequent microarousals, poor sleep quality and daytime drowsiness. An obstructed airway caused by an SDB event leads to a negative thoracic pressure during inspiration. This negative pressure impedes the cardiac preload and afterload. The cardiac system compensates by a change of the systemic and pulmonary blood pressure and an increased sympathetic activity, which contributes to cardiac arrhythmias. Both the change in preload and afterload, and the increased sympathetic outflow is considered as a major cause in hypertension (HTN). In addition, the frequent arousals and sleep deprivation due to periodic asphyxia also result in sympathetic nerve activation and contribute to hypertension.

Hypertension, which is also known as high blood pressure, is defined as the presence of a chronic elevation of systolic arterial pressure above 140 mm Hg or diastolic pressure above 90 mm Hg, see article "The Association of Obstructive Sleep Apnea and Hypertension", by Patel et.al, Cureus 11(6), June 7, 2019).

### SUMMARY OF THE INVENTION

It is an objective of the invention to provide control of a respiratory support device to treat sleep disordered breathing, while also improving hypertension.

According to a first aspect, there is provided a computer program product, comprising instructions which, when executed by a processing unit, cause the processing unit to carry out a method for controlling a respiratory support device to provide a pressurized airflow to a subject. The method comprises the steps of receiving a respiratory signal representative of respiratory information of the subject, determining at least one of a hypoxia and a hypercapnia based on the respiratory signal, and providing a control signal to the respiratory support device in response to determining the at least one of a hypoxia and a hypercapnia.

The respiration of a subject changes when the subject starts suffering from a lack of oxygen in the blood stream, i.e., hypoxia, or from an excess amount of carbon dioxide in the blood stream, i.e., hypercapnia. In case the subject suffers from hypoxia, chemoreceptors in the central arteries of the subject's body sense the lack of oxygen in the blood stream. As a result, the body causes a sympathetic activity that causes predominantly a change in breathing rate without substantially changing the tidal volume. The sympathetic activity is a stress reaction in an attempt to increase the oxygen level in the blood stream. In case the subject suffers from hypercapnia, chemoreceptors in the brain sense the excess carbon dioxide. As a result, the body causes a sympathetic activity that causes an increased work of breathing. This means deeper breaths i.e., a higher tidal volume, without changing the breathing rate. The sympathetic activity is a stress reaction in an attempt to decrease the carbon dioxide level in the blood stream. By receiving the respiratory signal, it is possible to determine whether the subject suffers from hypoxia and/or hypercapnia. By providing the control signal to the respiratory support device in response to determining the hypoxia and/or the hypercapnia, the respiratory support device is able to provide an improved treatment to reduce the hypoxia and/or hypercapnia. By this improved treatment, the sympathetic activity is reduced. Because the sympathetic activity is caused by the sympathetic nervous system, that also regulates blood pressure, the reduced sympathetic activity reduces hypertension. As a result, the method for controlling a respiratory support device provides the treatment for the SDB as well as helps to reduce hypertension.

According to a second aspect of the invention, there is provided a respiratory support device for providing a pressurized airflow to a subject. The respiratory support device comprises a processing unit configured to execute the computer program product of the first aspect. The respiratory support device comprises a sensor interface configured to communicate with at least one sensor to provide the respiratory signal from the at least one sensor to the processing unit, an airflow source to generate the pressurized airflow under control of the control signal, and a patient interface to provide the pressurized airflow to the subject.

According to a third aspect of the invention, there is provided a method for providing a further desired property for a further respiratory support device to provide a further pressurized airflow with the further desired property to a further subject. The method comprises receiving, from the respiratory support device according to the second aspect that carried out with the processing unit the computer program product according to the first aspect, a correlation signal representative of at least one of the first correlation, the second correlation and the third correlation. The method comprises receiving a further blood pressure signal representative of a blood pressure of the further subject, and determining the further desired property of the further pressurized airflow based on correlation signal and the further blood pressure signal.

In the third aspect, the correlation signal is received. The correlation signal is representative of at least one of a first correlation between a setting of the respiratory support device and the blood pressure, a second correlation between the setting of the respiratory support device and the respiratory information, and a third correlation between the setting of the respiratory support device and the medication use. The information from one or more of the correlations from one patient is used to determine the desired property of the further pressurized airflow for another subject. To optimize the further pressurized airflow for the other subject, the blood pressure of the other subject is taken into account when determining the desired property of the further pressurized airflow for the other subject. This way, information is used about how blood pressure is influenced by the setting of the respiratory support device, how the setting of the respiratory support device influences the respiration, and how medication use influences the blood pressure and respiration. With this information, the desired property of the pressurized airflow for the other subject is determined. This allows of an improved treatment for the other subject, because less time is needed to find which settings are desired for the other subject.

According to a fourth aspect of the invention, there is provided a data-processing system, configured to perform the method of the third aspect. The data-processing system comprises an input interface, a processing unit and an output interface. The input interface is configured to receive the correlation signal and the further blood pressure signal. The processing unit is configured to determine the further desired property based on the correlation signal and the further blood pressure signal. The output interface is configured to output an output signal representing the further desired property.

According to a fifth aspect of the invention, there is provided a computer-readable storage medium comprising the computer program product of the first aspect.

According to a sixth aspect of the invention, there is provided a method comprising receiving the respiratory signal representative of respiratory information of the subject, determining at least one of a hypoxia and a hypercapnia based on the respiratory signal, and providing a control signal to the respiratory support device in response to determining the at least one of a hypoxia and a hypercapnia.

According to a seventh aspect of the invention, there is provided a processing unit configured for controlling the respiratory support device according to the second aspect, configured to carry out the method of the first aspect.

### BRIEF DESCRIPTION OF THE DRAWINGS

Exemplary embodiments will now be described, by way of example only, with reference to the following figures, in which:
FIG. 1 depicts a first embodiment according to the invention;
FIG. 2 depicts tidal volume and breathing rate over time in case of hypoxia;
FIG. 3 depicts tidal volume and breathing rate over time in case of hypercapnia;
FIG. 4 depicts a method according to a second embodiment of the invention;
FIG. 5 depicts a data-processing system according to a third embodiment of the invention;
FIG. 6 depicts a method according to a fourth embodiment of the invention.

### DETAILED DESCRIPTION OF EMBODIMENTS

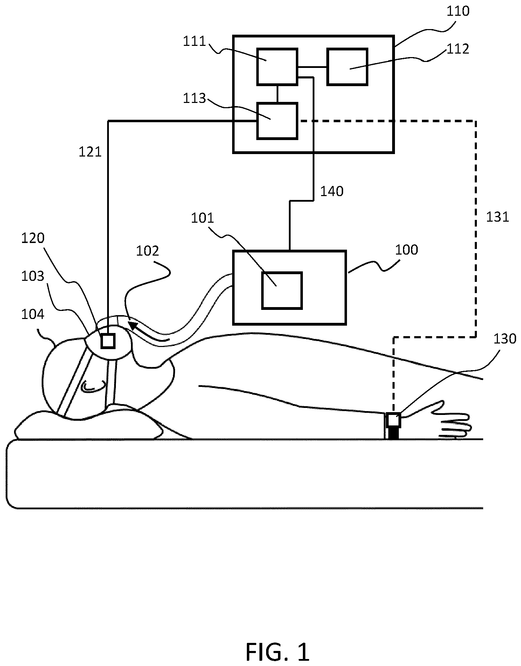

FIG. 1 depicts a first embodiment according to the invention. A respiratory support device 100 is for providing a pressurized airflow 102 to a subject 104. There is provided a processing unit 100 comprising a sensor interface 113. The respiratory support device 100 comprises an airflow source 101, and a patient interface 103. The processing unit 110 is configured to execute a computer program product as described further below. The sensor interface 113 is configured to communicate with at least one sensor 120 to provide the respiratory signal 121 from the at least one sensor 120 to the processing unit 110. The airflow source 101 is to generate the pressurized airflow 102 under control of a control signal 140. The patient interface 103 is adapted to provide the pressurized airflow 102 to the subject 104. In this embodiment, the at least one sensor 120 is a respiratory sensor, such as a flow sensor or a pressure sensor. The sensor interface 113 is configured to communicate with the respiratory sensor 120 to receive the respiratory signal 121.

The processing unit 110 is configured to execute the computer program product. The computer program product comprises instructions which, when executed by the processing unit 110, cause the processing unit 110 to carry out a method for controlling the respiratory support device 100 to provide the pressurized airflow 102 to a subject 104. The method comprising the steps of receiving a respiratory signal 121 representative of respiratory information of the subject 104, determining at least one of a hypoxia and a hypercapnia based on the respiratory signal 121, and providing the control signal 140 to the respiratory support device 100 in response to determining the at least one of a hypoxia and a hypercapnia.

The processing unit 110 is configured to perform the data processing. The processing unit 110 can be implemented in numerous ways, with software and/or hardware, to perform the various functions required. The processing unit 110 employs, for example one or more microprocessors 111 that are programmed using software (e.g. microcode) to perform the required functions. The processing unit 110 is, for example, implemented as a combination of dedicated hardware to perform some functions and one or more programmed microprocessors and associated circuitry to perform other functions.

Examples of circuitry that may be employed in the processing unit 110 include, but are not limited to, conventional microprocessors, application specific integrated circuits (ASICs), and fieldprogrammable gate arrays (FPGAs). Thus, the processing unit 110 may be embodied as a digital and/or analog processing system. Functions implemented by a processing unit 110 may be implemented with a single processor 111 or by multiple separate processors which together be considered to constitute a "processing unit 110".

The sensor 120 that generates the respiratory signal 121 is, for example, an air pressure sensor or an air flow sensor. For example, the sensor 120 is arranged in the housing of the respiratory support device 100. For example the sensor 120 is an acceleration sensor that provides the respiratory signal 121 based on movement of the subject 104. For example, the acceleration sensor is located on the chest or the abdomen of the subject 104. The acceleration sensor generates the respiratory signal 121 based on movement due to expansion and retraction of the chest and/or abdomen.

For example, hypoxia has a decrease of mean oxygen saturation of >4%. For example, hypercapnia has an elevation in the partial pressure of carbon dioxide above 45 mm Hg.

For example, the sensor interface 113 has a socket to receive the sensor 120. The sensor 120 has a plug to cooperate with the socket. For example, the sensor interface 113 provides wireless communication with the sensor 120. For example, the wireless communication comprises a Bluetooth connection, or a Wi-Fi connection, or a connection using radio waves.

For example, the airflow source 101 comprises a blower unit. The blower unit comprises an impeller and a motor. The motor is coupled to the impeller and adapted to rotate the impeller. The impeller is adapted to displace air when rotated by the motor. By displacing air, the impeller creates the pressurized airflow 102. The control signal 140 controls the speed, i.e., the rotations per minute, of the motor to adjust the pressurized air. For example, increasing the motor speed increases the pressure of the pressurized air.

The patient interface 103 comprises a mask to provide the pressurized airflow 102 to the mount and/or the nose of the subject 104. For example, the patient interface 103 comprises a face mask or a nasal mask or a mouth mask. For example, the patient interface 103 comprises a hose to connect the mask to the respiratory support device 100.

Optionally, the processing unit 110 comprises a memory 112. For example, the memory 112 stores the computer program product. For example, the memory stores data relating to the respiratory signal 121 and/or data relating to the control signal 140.

In an embodiment, the respiratory support device 100 is a PAP device, such as a CPAP device, a BiPAP device or an auto-PAP device.

In FIG. 1 the processing unit 110 is depicted as a unit that is separated from the respiratory support device 100. In an embodiment, the processing unit 110 and the respiratory support device 100 are integrated in a single unit.

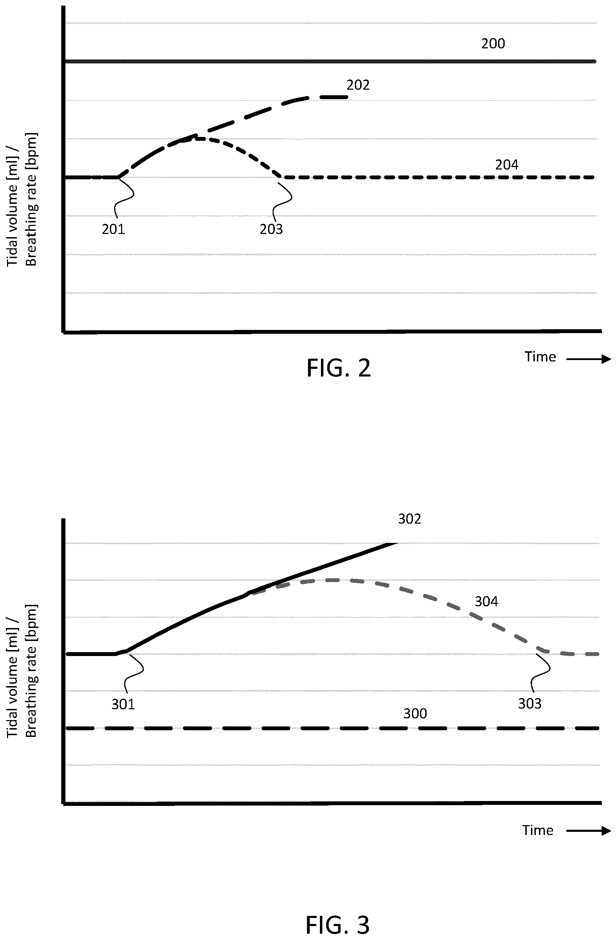

FIG. 2 and FIG. 3 depict the tidal volume and the breathing rate over time in case of hypoxia and in case of hypercapnia respectively. The tidal volume during normal breathing is, for example, in the range of 570 - 780 ml for an adult male. The tidal volume during normal breathing is, for example, in the range of 500 - 550 ml for an adult female. The tidal volume is, for example, about 7 ml/kg of bodyweight of the subject 104. The breathing rate for normal breathing is, for example, in the range of 12 to 20 breaths per minute (bpm) or in the range of 16-18 bpm. Normal breathing is breathing when the subject 104 does not have hypoxia or hypercapnia. It is possible that the subject 104 has a condition that causes the tidal volume during normal breathing to be lower or higher than the tidal volume of an average healthy adult without that condition. It is possible that the subject 104 has a condition that causes the breathing rate during normal breathing to be lower or higher than the breathing rate of an average healthy adult without that condition. Even with such a condition, the tidal volume and the breathing rate may vary due to hypoxia or hypercapnia as indicated in FIG. 2 and FIG. 3. The absolute values of the tidal volume and the breathing rate depend on many factors, such as gender, age, length, and bodyweight of the subject 104, and on any medical conditions the subject 104 has. Because of the differences in absolute values of the tidal volume and the breathing rate between different subjects, FIG. 2 and FIG. 3 does not have values on the axis.

FIG. 2 depicts the tidal volume and the breathing rate overtime in case of hypoxia. Line 200 schematically indicates the tidal volume of the subject 104 overtime in case the subject 104 suffers from hypoxia. While suffering from hypoxia, the tidal volume of the subject 104 remains unchanged, or changes only in a limited way, for example not significantly different to changes of the tidal volume in case the subject 104 has no hypoxia. As a result of the hypoxia, the body causes a sympathetic activity that causes an increase in the breathing rate, as is indicated with point 201 on line 202. The sympathetic activity is aimed at increasing the level of oxygen in the blood by breathing faster. The sympathetic activity, however, also results in an increased blood pressure, which negatively affects a subject 104 suffering from hypertension. Based on respiratory information, the processing unit 110 determines that the subject 104 suffers from hypoxia. In response, the processing unit 110 provides the control signal 140 to the respiratory support device 100. The respiratory support device 100 adjusts the pressurized airflow 102 to improve the level of oxygen in the blood of the subject 104. As a result of the adjusted pressurized airflow 102, the hypoxia is reduced, causing the breathing rate to return to a normal rate, as indicated by point 203 of line 204. The normal breathing rate is, for example, the same or almost the same as the breathing rate as before the hypoxia. For example, the differences between the breathing rate before and after the hypoxia are within a range of variations of the breathing rate of a healthy respiration.

In an embodiment, the respiratory information comprises information about a variability in a breathing rate of the subject 104. The method comprises determining hypoxia based on the variability in the breathing rate. So based on the increase in the breathing rate as indicated by line 202, the respiratory support device 100 is able to adjust the pressurized airflow 102 to the subject 104 to improve the oxygen level in the blood. As a result, the breathing rate of the subject 104 returns to the value before the hypoxia, as is indicated by point 203 on line 204. The variability of the breathing rate comprises, for example, a change in an average breathing rate over the previous 1 minute or 5 minutes or 15 minutes. The variability of the breathing rate comprises, for example, a change of an average breathing rate over the previous 3 or 5 or 10 breathing cycles. The variability of the breathing rate comprises, for example, a change of a timing of one breathing cycle compared to another breathing cycle. The variability of the breathing rate comprises, for example, a change in one or more of the four phases of breathing. The four phases of breathing are, in chronological order, the inhalation phase, the suspension phase, the exhalation phase and the rest phase. The variability of the breathing rate comprises, for example, a gradual increase of the breathing rate over more than 3 or more than 5 or more than 10 breathing cycles. Such gradual increase may be indicative of hypoxia, whereas a sudden increase in the breathing rate may be indicative of another type of event, like an arousal of the subject 104 or an SDB event.

In an embodiment, the method comprises, in response to determining hypoxia, providing the control signal 140 to the respiratory support device 100 to cause the pressurized airflow 102 to support an increase of the breathing rate of the subject 104. When determining the subject 104 is suffering from hypoxia, the respiratory support device 100 is able to adjust the pressurized airflow 102 to support the increase of the breathing rate of the subject 104. For example, the pressurized airflow 102 has a high pressure during the inhalation phase and a low pressure during the exhalation phase. To support the increase the breathing rate of the subject 104, the respiratory support device 100, for example, changes the time period of pressure support during the inhalation phase, and changes the duration of pressure support during the exhalation phase to trigger the subject to adapt the timely length of the breathing cycle. The subject 104 remains in spontaneous breathing but the respiratory support device 100 paces the spontaneous breathing to increase the breathing rate, by changing the duration of the pressure support during the inhalation phase and/or exhalation phase. By pacing the breathing, the respiratory support device 100 adapts the breathing rate smoothly. For example, the respiratory support device 100 reduces the time between starting the inhalation phase and ending the inhalation phase. For example, the respiratory support device 100 reduces the time between starting the exhalation phase and ending the exhalation phase. This means that the pressurized airflow 102 changes between the high pressure and the low pressure more frequently. As a result, the increase of the breathing rate is supported by the respiratory support device 100, cause the hypoxia to be reduced. For example, breathing rate is reduced by 100 ms or 200 ms per breathing cycle.

FIG. 3 depicts the tidal volume and the breathing rate overtime in case of hypercapnia. Line 300 schematically indicates the breathing rate of the subject 104 over time in case the subject 104 suffers from hypercapnia. While suffering from hypercapnia, the breathing rate of the subject 104 remains unchanged, or changes only in a limited way, for example not significantly different to changes of the breathing rate in case the subject 104 has no hypercapnia. As a result of the hypercapnia, the body causes a sympathetic activity that causes an increase in the tidal volume, as is indicated with point 301 on line 302. The sympathetic activity is aimed at decreasing the level of carbon dioxide in the blood by taking deeper breaths. The sympathetic activity, however, also results in an increased blood pressure, which negatively affects a subject 104 suffering from hypertension. Based on respiratory information, the processing unit 110 determines that the subject 104 suffers from hypercapnia. In response, the processing unit 110 provides the control signal 140 to the respiratory support device 100. The respiratory support device 100 adjusts the pressurized airflow 102 to increase the volume of the inflowing air during the inhalation phase and to increase the outflowing air in the exhalation phase, which consequently decreases the level of carbon dioxide in the blood of the subject 104. As a result of the adjusted pressurized airflow 102, the hypercapnia is reduced, causing the tidal volume to return to a normal rate, as indicated by point 303 of line 404. The normal tidal volume is, for example, the same or almost the same as the tidal volume as before the hypercapnia. For example, the differences between the tidal volume before and after the hypercapnia are within a range of variations of the tidal volume of a healthy respiration.

In an embodiment, the respiratory information comprises information about a variability in a tidal volume of the subject 104. The method comprises determining hypercapnia based on the variability in the tidal volume. So based on the increase in the tidal volume as indicated by line 302, the respiratory support device 100 is able to adjust the pressurized airflow 102 to the subject 104 to support the decrease of the carbon dioxide level in the blood. As a result, the tidal volume of the subject 104 returns to the value before the hypercapnia, as is indicated by point 303 on line 304. The variability of the tidal volume comprises, for example, a change of an average tidal volume over the previous 1 minute or 5 minutes. The variability of the tidal volume comprises, for example, the tidal volume exceeding a threshold value for one or more breathing cycles, such as 3 breathing cycles or 5 breathing cycles. The variability of the tidal volume comprises, for example, a gradual increase of the tidal volume over more than 3 or more than 5 or more than 10 breathing cycles. Such gradual increase may be indicative of hypercapnia, whereas a sudden increase in the tidal volume may be indicative of another type of event, like an arousal of the subject 104 or an SDB event.

In an embodiment, the method comprises, in response to determining hypercapnia, providing the control signal 140 to the respiratory support device 100 to cause the pressurized airflow 102 to support an increase of the tidal volume of the subject 104. When determining the subject 104 is suffering from hypercapnia, the respiratory support device 100 is able to adjust the pressurized airflow 102 to support the increase of the tidal volume of the subject 104. For example, the pressurized airflow 102 has an increased pressure during the inhalation phase to help the subject 104 to take deeper inhales. For example, the pressurized airflow 102 has a decreased pressure during the exhalation phase to help the subject 104 to take deeper exhales. As a result, the increase of the tidal volume is supported by the respiratory support device 100, causes the hypercapnia to be reduced. For example, the pressure during the inhalation phase is increased by 0,5 or 1,0 cmH20. For example, the pressure during the exhalation phase is decreased by 0,5 or 1,0 cmH2O. For example, in case the subject 104 does not suffer from hypercapnia, the pressure during the inhalation phase is in the range of 4 - 24 cmH2O, for example around 9 cmH2O. For example, in case the subject 104 does not suffer hypercapnia, the pressure during the exhalation phase is lower than or equal to the pressure during the exhalation phase. For example, the pressure during the exhalation phase is in the range of 4 - 16 cmH2O.

In an embodiment, the method comprises receiving a blood pressure signal 131 representative of a blood pressure of the subject 104, as depicted in FIG. 1. The method comprises determining a first correlation between a setting of the respiratory support device 100 and the blood pressure. The setting causes the respiratory support device 100 to provide the pressurized airflow 102 with a desired property. The method comprises providing the control signal 140 based on the first correlation.

The subject 104 wears a wearable blood pressure sensor 130. The blood pressure sensor 130 generates the blood pressure signal 131 representative of the blood pressure of the subject 104. For example, the blood pressure sensor 130 is a photoplethysmographic (PPG) sensor, or an electronic sphygmomanometer, or a tonometric sensor, or a blood pressure cuff, or a pulse wave velocity (PWD) measurement sensor, or a pulse-arrival-time (PAT) sensor. FIG. 1 indicates the blood pressure sensor 130 is worn on the wrist. Alternatively, the blood pressure sensor 130 is integrated in the patient interface 103. Alternatively, the blood pressure sensor 130 is configured to be placed at a different part of the body, such as at a fingertip or the chest or the upper arm. For example, multiple sensors provide signals representing the blood pressure. For example, the blood pressure sensor 130 is configured to communicate with the respiratory support device 100 via a wire or wireless, for example via Bluetooth or Wi-Fi or radio waves.

As described above, there is a correlation between the respiration of the subject 104, which may cause an activation of the sympathetic system, and the blood pressure that is regulated by the same sympathetic system. The first correlation between the setting of the respiratory support device 100 and the blood pressure gives an insight about how the respiratory support device 100 affects the blood pressure of the subject 104. Based on the first correlation, it is possible to control the respiratory support device 100 to improve the blood pressure. For example, in case a change of the setting of the respiratory support device 100 results in a worsening of the hypertension, the respiratory support device 100 is controlled to undo the change of the setting, to make a further change of the setting, or to change another setting. For example, the change of the setting is a decrease of the pressure of the pressurized airflow 102. The pressure is decreased because the apnea hypopnea index (AHI) of the subject 104 allows for a lower pressure. However, the lower pressure results in a worsening of the hypertension. The worsening of the hypertension is determined based on the blood pressure signal 131. In response, the respiratory support device 100 is controlled to increase the pressure of the pressurized airflow 102 to reduce the hypertension. For example, the first correlation is based on the blood pressure signal 131 of the current sleep session, or from multiple sleep sessions.

The setting comprises, for example, a maximum pressure of the pressurized airflow 102, a minimum pressure of the pressurized airflow 102, a pressure profile of the pressurized airflow 102, a pressure gradient of the pressurized airflow 102, a timing of a change in pressure of the pressurized airflow 102, a temperature of the pressurized airflow 102, and/or a humidity of the pressurized airflow 102.

In an embodiment, the blood pressure signal 131 is representative of a blood pressure of the subject 104 while the subject 104 is not affected by the pressurized airflow 102.

In this embodiment, the blood pressure signal 131 is representative of the blood pressure of the subject 104 while the subject 104 is not using the respiratory support device 100. For example, the subject 104 uses a wearable blood pressure sensor 130 during the day. The wearable blood pressure sensor 130 collects data of the blood pressure during the day. Then, the wearable blood pressure sensor 130 transfers the collected data as the blood pressure signal 131 to the processing unit 110, for example, while the subject 104 sets up the respiratory support device 100 just before going to sleep. For example, the wearable blood pressure sensor 130 transfers the collected data as the blood pressure signal 131 to the processing unit 110 via a mobile phone and the internet, or via Bluetooth. For example, the wearable blood pressure sensor 130 requires docking with the respiratory support device 100 to transfer the data as the blood pressure signal 131 to the processing unit 110. For example, the blood pressure sensor 130 collects data of the blood pressure continuously through the day, or only at a limited number of moments.

The effect of the pressurized airflow 102 provided by the respiratory support device 100 during sleep has an effect on the blood pressure of the subject 104 during the day. So even if the subject 104 has not been using the respiratory support device 100 for several hours, the effect on the blood pressure is still present. So by measuring the blood pressure while the subject 104 is not using the respiratory support device 100, it is possible to determine whether the setting of the respiratory support device 100 helps to reduce hypertension, or whether an adjustment of the setting is desired.

In an embodiment, the method comprises determining a trend over time between the pressurized airflow 102 provided to the subject 104 and the blood pressure signal 131. The method comprises determining the desired property for the pressurized airflow 102 is based on the trend.

The pressurized airflow 102 provided to the subject 104 has an effect on the blood pressure, but it may take several days or weeks before this effect becomes apparent. Meanwhile, variations of the blood pressure may occur, which are not related to the use of the respiratory support device 100. For example, the variations of the blood pressure may be caused by the daily activities performed by the subject 104, differences in diet (such as differences in salt intake), changes in psychological stress, or drinking alcohol. By determining a trend over time, the influence of variations of the blood pressure which are not related to the use of the respiratory support device 100 is reduced. This allows more accurate control of the respiratory support device 100.

In an embodiment, the respiratory information comprises information about at least one of a sleep disordered breathing (SDB) event, obstructive sleep apnea (OSA), central sleep apnea (CSA), obesity hypoventilation syndrome (OHS), and COPD. The method comprises determining a second correlation between the setting of the respiratory support device 100 and the respiratory information, and providing the control signal 140 based on the second correlation.

An SDB event is an event in which the normal breathing of a sleeping subject 104 is disturbed, for example characterized by snoring and/or an increased respiratory effort. OSA is characterized by episodes of a complete or partial collapse of the upper airway, causing a decrease in oxygen saturation or arousal from sleep. CSA is characterized by a cessation of airflow with alterations in the respiration effort. Obesity hypoventilation syndrome is a condition in which severely overweight people fail to breathe rapidly or deeply enough, resulting in low oxygen levels and high carbon dioxide levels in the blood. Chronic obstructive pulmonary disease, COPD, is a type of progressive lung disease characterized by long-term respiratory symptoms and airflow limitation.

The respiratory support device 100 may be used to support the breathing of a subject 104 suffering from one or more of these conditions. To assess the efficacy of the support, the respiratory information obtained by the respiratory support device 100 has information about the condition. For example, the information includes information about the apnea-hypopnea index, AHI, the number of breathing stops, the amount or severity of coughing, the volume of inhaled air per unit of time, and/or the composition of exhaled air, such as the amount of carbon dioxide in the exhaled air.

By determining the second correlation, it is possible to determine how a setting of the respiratory support device 100 affects the condition. Based in the second correlation, it is possible to quickly determine which setting is to be used, in case the subject 104 suffers from such a condition in a certain amount. By using the second correlation, less time is needed to find a setting that works sufficiently for a subject 104. For example, in case the condition of the subject 104 changes, using the second correlation helps to quickly find an adjusted setting to support the changed condition.

In an embodiment, the method comprises receiving a medication signal representative of information of a medication use by the subject 104. The method comprises determining a third correlation between the setting of the respiratory support device 100 and the medication use. The method comprises providing the control signal 140 based on the third correlation.

Some medications have an influence on the respiration of a subject 104. Some of these medications are prescribed to influence the respiration, whereas other medications have one or more side effects that influence the respiration. By using the information of the medication use and correlating that to the setting of the respiratory support device 100, it is possible to determine the third correlation. The third correlation provides information about how the medication use influences the respiration. For example, it is possible to decrease the pressure setting of the respiratory support device 100 in case the third correlation indicates that a medication has a positive effect on the respiration. For example, it is possible to increase the pressure setting of the respiratory support device 100 in case the third correlation indicates that a medication has a negative effect on the respiration. The third correlation takes, for example, into account, the type of medication, the dose, the time the medication is already being used, the time of the most recent dose, or a combination of medications.

For example, the medication signal is generated by a user interface. The subject 104, or the professional caretaker, provides information about the medication via the user interface. For example, the user interface is an app on a mobile device, which allows information about the medication to be filled in. In another example, the medication signal is generated by a sensor. The sensor detects when the subject 104 takes a medication. For example, when the subject 104 inhales the medication, or when a pump provides the medication in infusion therapy.

In an embodiment, the method comprises providing an initial control signal to the respiratory support device 100 to provide the pressurized airflow 102 with an initial property before providing the control signal 140. The respiratory information comprises information about a respiration of the subject 104 while affected by the pressurized airflow 102 with the initial property.

In this embodiment, the subject 104 starts using the respiratory support device 100 under the initial control signal. The pressurized airflow 102 has an initial property as a result of the initial control signal. The initial property is, for example, a default property that is a suitable property for most subjects or for most subjects with the same condition as the subject 104 or for most subjects with the same body weight as the subject 104. The pressurized airflow 102 with the initial property may already bring some benefit to the subject 104. However, the benefit may not be sufficient for effective support of the subject 104. While the subject 104 is being provided with the pressurized airflow 102 with the initial property, the respiratory information is obtained. Based on the respiratory information, it is obtained whether the subject 104 suffers from hypoxia or hypercapnia. Depending on the determination whether the subject 104 suffers from hypoxia or hypercapnia, the setting of the respiratory support device 100 is adjusted to provide the pressurized airflow 102 with the desired property. By providing the pressurized airflow 102 first with the initial property and then with the desired property, it is possible to provide at least some support to the subject 104 quickly, while determining the desired property for the optimized support.

In an embodiment, the method comprises providing a plurality of initial control signals to the respiratory support device 100 to provide a plurality of pressurized airflows before providing the control signal 140. Each of plurality of pressurized airflows has a different initial property. For each of the plurality of initial control signals, the method comprises receiving a blood pressure signal 131 representative of a blood pressure of the subject 104 while affected by the corresponding pressurized airflow 102, and receiving a respiratory signal 121 representative of respiratory information of the subject 104 while affected by the corresponding pressurized airflow 102. The method further comprises selecting one of the plurality of pressurized airflows as the desired pressurized airflow 102 based on the blood pressure signal 131 and the respiratory signal 121 corresponding to the one of the plurality of initial pressures. The method comprises providing the control signal 140 based on the initial control signal corresponding to the desired pressurized airflow 102.

The relation between the pressurized airflow 102 and the blood pressure of a subject 104 may depend on individual characteristics of the subject 104, such as age or bodyweight or gender or medical conditions. To improve the time to find a desired property of the pressurized airflow 102, the subject 104 is first provided with a plurality of pressurized airflows under control of the initial control signals. The plurality of pressurized airflows have initial properties, for example, that provide good support to subjects with the same age, bodyweight or medical condition as the subject 104 receiving the pressurized airflow 102. The plurality of pressurized airflows have initial properties, for example, divided over a range of properties suitable for the subject 104. For example, the range of properties is a range of pressures of the pressurized airflow 102, ranging from a minimum pressure to a maximum pressure. For example, the range of properties is a range of timing between a high pressure and a low pressure of the pressurized airflow 102, ranging from a minimum timing to a maximum timing. For each of the initial properties, the blood pressure signal 131 is determined. Based on the outcome of the blood pressure signal 131, one of the initial properties is selected as the desired property. For example, the initial property is selected as the desired property based on the blood pressure signal 131 and the respiratory signal 121. For example, the blood pressure signal 131 indicates the largest reduction of the hypertension compared to the blood pressure signals corresponding to the pressurized airflows with the other initial properties.

For example, one initial control signal is provided to the respiratory support device 100 for a certain amount of time, after which a following initial control signal is provided to the respiratory support device 100. For example, the certain amount of time is 30 minutes or 1 hour or 2 hours or 4 hours. For example, one initial control signal is provided to the respiratory support device 100 for a whole sleep session, whereas a following initial control signal is provided to the respiratory support device 100 for the whole next sleep session. For example, one or more initial control signals are used multiple times. For example, an initial control signal is used as a default control signal and is used in between providing the other initial control signals. The default control signal helps to bring the subject 104 into a default state, which allows to better compare the outcomes of the different initial control signals. For example, the default control signal helps to bring the blood pressure of the subject 104 to a default value, before providing a next initial control signal.

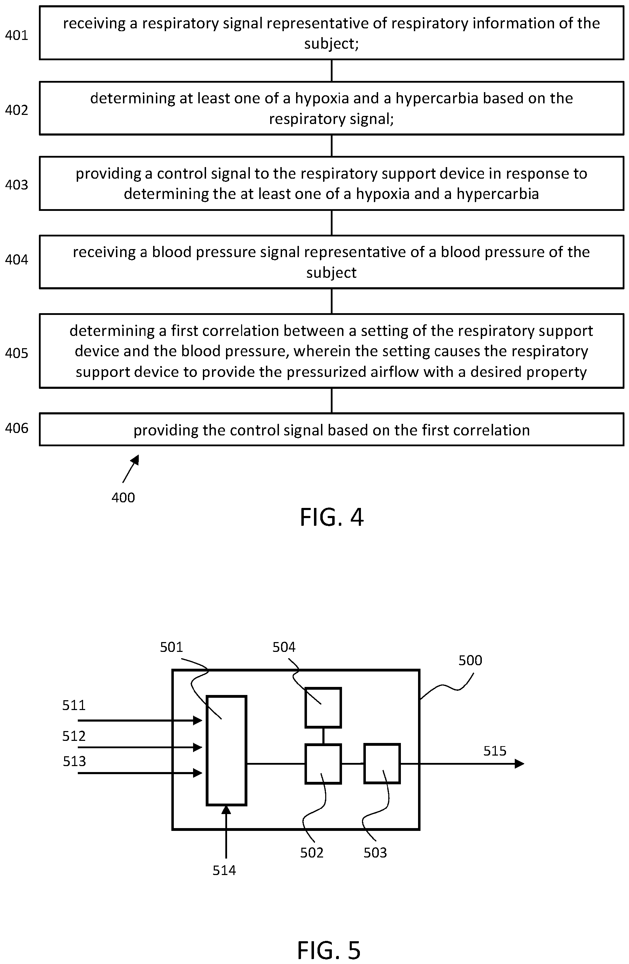

FIG. 4 depicts a method 400 according to a second embodiment of the invention. For example, the method 400 is carried out by the processing unit 110 to control the respiratory support device 100 to provide a pressurized airflow 102 to a subject 104. For example, a computer program product is provided, comprising instructions which, when executed by the processing unit 110, cause the processing unit 110 to carry out the method 400 for controlling a respiratory support device 100 to provide a pressurized airflow 102 to a subject 104. For example, there is provided a computer-readable storage medium comprising the computer program product. For example, there is provided a processing unit 110 for controlling a respiratory support device 100 according to the first embodiment, configured to carry out the method 400 according to the second embodiment.

The method 400 comprises steps 401-403. Step 401 comprises receiving the respiratory signal 121 representative of respiratory information of the subject 104. Step 402 comprises determining at least one of a hypoxia and a hypercapnia based on the respiratory signal 121. Step 403 comprises providing a control signal 140 to the respiratory support device 100 in response to determining the at least one of a hypoxia and a hypercapnia.

Optionally, the respiratory information comprises information about a variability in a breathing rate of the subject 104. The method 400 comprises determining hypoxia based on the variability in the breathing rate.

Optionally, the method 400 comprises, in response to determining hypoxia, providing the control signal 140 to the respiratory support device 100 to cause the pressurized airflow 102 to support an increase of the breathing rate of the subject 104.

Optionally, the respiratory information comprises information about a variability in a tidal volume of the subject 104. The method 400 comprises determining hypercapnia based on the variability in the tidal volume.

Optionally, the method 400 comprises, in response to determining hypercapnia, providing the control signal 140 to the respiratory support device 100 to cause the pressurized airflow 102 to support an increase of the tidal volume of the subject 104.

Optionally, the method 400 comprises steps 404-406. Step 404 comprises receiving a blood pressure signal 131 representative of a blood pressure of the subject 104. Step 405 comprises determining a first correlation between a setting of the respiratory support device 100 and the blood pressure. The setting causes the respiratory support device 100 to provide the pressurized airflow 102 with a desired property. Step 406 comprises providing the control signal 140 based on the first correlation.

Optionally, the blood pressure signal 131 is representative of a blood pressure of the subject 104 while the subject 104 is not affected by the pressurized airflow 102.

Optionally, the method 400 comprises determining a trend over time between the pressurized airflow 102 provided to the subject 104 and the blood pressure signal 131. The method 400 comprises determining the desired property for the pressurized airflow 102 is based on the trend.

Optionally, the respiratory information comprises information about at least one of a sleep disordered breathing (SDB) event, obstructive sleep apnea (OSA), central sleep apnea (CSA), obesity hypoventilation syndrome, and COPD. The method 400 comprises determining a second correlation between the setting of the respiratory support device 100 and the respiratory information, and providing the control signal 140 based on the second correlation.

Optionally, the method 400 comprises receiving a medication signal representative of information of a medication use by the subject 104, determining a third correlation between the setting of the respiratory support device 100 and the medication use, and providing the control signal 140 based on the third correlation.

Optionally, the method 400 comprises providing an initial control signal to the respiratory support device 100 to provide the pressurized airflow 102 with an initial property before providing the control signal 140. The respiratory information comprises information about a respiration of the subject 104 while affected by the pressurized airflow 102 with the initial property. Further optionally, the method 400 comprises providing a plurality of initial control signals to the respiratory support device 100 to provide a plurality of pressurized airflows before providing the control signal 140. Each of plurality of pressurized airflows has a different initial property. For each of the plurality of initial control signals, the method 400 comprises receiving a blood pressure signal 131 representative of a blood pressure of the subject 104 while affected by the corresponding pressurized airflow 102, and receiving a respiratory signal 121 representative of respiratory information of the subject 104 while affected by the corresponding pressurized airflow 102. The method comprises selecting one of the plurality of pressurized airflows as the desired pressurized airflow 102 based on the blood pressure signal 131 and the respiratory signal 121 corresponding to the one of the plurality of initial pressures, and providing the control signal 140 based on the initial control signal corresponding to the desired pressurized airflow 102.

FIG. 5 depicts a data-processing system 500 according to a third embodiment of the invention. The data-processing system 500 comprises an input interface 501, a processing unit 502 and an output interface 503. The input interface 501 is configured to receive from the respiratory support device 100 according to the first embodiment at least one of a first correlation signal 511, a second correlation signal 512 and a third correlation signal 513. The first correlation signal 511 is representative of the first correlation. The second correlation signal 512 is representative of the second correlation. The third correlation signal 513 is representative of the third correlation. For example, the input interface 501 is configured to receive a correlation signal representative of at least one of the first correlation, the second correlation and the third correlation. For example, the input interface 501 is configured to receive only the first correlation signal 511, only the first correlation signal 511 and the second correlation signal 512, only the first correlation signal 511 and the third correlation signal 513, or only the second correlation signal 512 and the third correlation signal 513.

The input interface 501 is configured to receive a further blood pressure signal 514 from a further respiratory support device or from a further blood pressure sensor. The further blood pressure signal 514 is representative of a blood pressure of a further subject. For example, the further respiratory support device is the same type of respiratory support device 100 as the first embodiment, but used by a different subject. For example, the further respiratory support device is of a different type than the first embodiment.

The processing unit 502 is configured to determine a further desired property based on the correlation signal and the further blood pressure signal 514. The output interface 503 is configured to output an output signal 515 representing the further desired property.

The processing unit 502 makes use of correlations that are already determined for the subject 104, to determine the desired property of the pressurized airflow 102 for the further subject. This reduces the time needed to determine the desired property of the pressurized airflow 102 for the further subject.

For example, both subject 104 and the further subject share one or more characteristics, such as age of gender or medical condition. For example, both subject 104 and the further subject suffer from OSA, CSA, obesity hypoventilation syndrome, or COPD. For example, both subject 104 and the further subject use the same medication. By making use of the first correlation, the second correlation and/or the third correction, the further subject 104 can be provided with the pressurized airflow 102 at the desired property, which provides an improved decrease of hypertension for the further subject.

Optionally, the data-processing system 500 comprises a memory 504. For example, the memory 504 stores software to operate the data-processing system 500. For example, the memory 504 stores a computer program product having instruction which, when executed by the data-processing system 500, cause the data-processing system 500 to carry out the method according to the fourth embodiment of the invention, as is described below. For example, the memory 504 stores data relating to the first correlation, the second correlation and/or the third correlation.

For example, the data-processing system 500 comprises a server connected to the internet. The respiratory support device 100 and the further respiratory support device are connected to the server via the internet. The further respiratory support device receives the output signal 515 via the internet and provides the pressurized airflow 102 with the desired property in correspondence with the output signal 515.

For example, the data-processing system 500 is integrated in the respiratory support device. The respiratory support device 100 and the further respiratory support device are each provided with a communication device to communicate with each other. Via the communication devices, the output signal 515 is transferred. For example, each of the respiratory support device 100 and the further respiratory support device comprise a data-processing system 500.

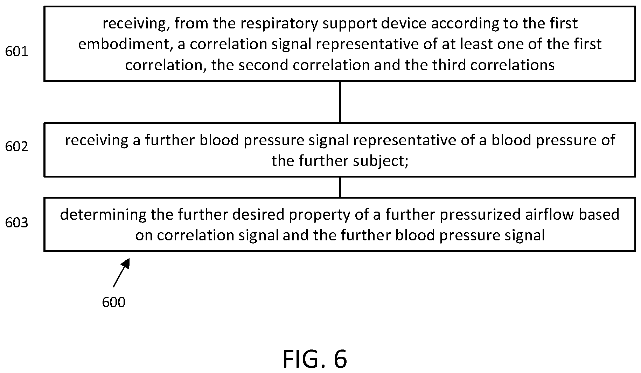

FIG. 6 depicts a method 600 according to a fourth embodiment of the invention. For example, the data-processing system 500 according to the third embodiment is configured to perform the method 600 according to the fourth embodiment. For example, there is provided a computer program product having instructions which, when executed by a processing unit 502, cause the processing unit 502 to carry out the method 600. For example, there is provided a computer-readable storage medium comprising the computer program product.

The method 600 is for providing a further desired property for a further respiratory support device to provide a further pressurized airflow with the further desired property to a further subject. The method 600 comprises receiving from the respiratory support device 100 according to the first embodiment a correlation signal representative of at least one of the first correlation, the second correlation and the third correlation, and receiving a further blood pressure signal 514 representative of a blood pressure of the further subject. The method 600 comprises determining the further desired property of the further pressurized airflow based on correlation signal and the further blood pressure signal 514.

Optionally, the further blood pressure signal 514 is received from the further respiratory support device.

In the various embodiments, the processing unit 110 and the processing unit 502 may be associated with the memory 112 and memory 504 respectively. Each or both of memory 112 and memory 504 include, for example, one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

The computer programs described in the various embodiments may be stored/distributed on a suitable medium, such as an optical storage medium or a solid-state medium supplied together with or as part of other hardware, but may also be distributed in other forms, such as via the Internet or other wired or wireless telecommunication systems.

The processing unit 110 and/or the processing unit 502 may execute a machine-learning algorithm. For example, the machine-learning algorithm is a self-training algorithm that processes input data from the respiratory signal and the blood pressure signal in order to produce the control signal 140. For example, the machine-learning algorithm is used to determine the trend over time between the pressurized airflow provided to the subject 104 and the blood pressure signal 131. For example, the machine-learning algorithm is a self-training algorithm that processes input data from the correlation signal 511-513 and the further blood pressure signal 514 in order to produce the further desired property. Examples of suitable machine-learning algorithms include decision tree algorithms and artificial neural networks. Other machine-learning algorithms such as logistic regression, support vector machines or Naive Bayesian models are suitable alternatives.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. Any reference signs in the claims should not be construed as limiting the scope.

It should be understood that the detailed description and specific examples, while indicating exemplary embodiments of the devices, units, systems, and methods, are intended for purposes of illustration only and are not intended to limit the scope of the invention. It should be understood that the Figures are merely schematic and are not drawn to scale. It should also be understood that the same reference numerals are used throughout the Figures to indicate the same or similar parts.

## Claims

1. A computer program product, comprising instructions which, when executed by a processing unit (110), cause the processing unit (110) to carry out a method (400) for controlling a respiratory support device (100) to provide a pressurized airflow (102) to a subject (104), the method (400) comprising the steps of:
receiving a respiratory signal (121) representative of respiratory information of the subject (104);
determining at least one of a hypoxia and a hypercapnia based on the respiratory signal (121);
providing a control signal (140) to the respiratory support device (100) in response to determining the at least one of a hypoxia and a hypercapnia.

2. The computer program product according to claim 1, wherein the respiratory information comprises information about a variability in a breathing rate of the subject (104), the method (400) comprising determining hypoxia based on the variability in the breathing rate.

3. The computer program product according to claim 1, wherein the method (400) comprises, in response to determining hypoxia, providing the control signal (140) to the respiratory support device (100) to cause the pressurized airflow (102) to support an increase of the breathing rate of the subject (104).

4. The computer program product according to any one of the preceding claims, wherein the respiratory information comprises information about a variability in a tidal volume of the subject (104), the method (400) comprising determining hypercapnia based on the variability in the tidal volume.

5. The computer program product according to any one of the preceding claims, wherein the method (400) comprises, in response to determining hypercapnia, providing the control signal (140) to the respiratory support device (100) to cause the pressurized airflow (102) to support an increase of the tidal volume of the subject (104).

6. The computer program product according to any one of the preceding claims, wherein the method (400) comprises receiving a blood pressure signal (131) representative of a blood pressure of the subject (104);
determining a first correlation between a setting of the respiratory support device (100) and the blood pressure, wherein the setting causes the respiratory support device (100) to provide the pressurized airflow (102) with a desired property;
providing the control signal (140) based on the first correlation.

7. The computer program product according to claim 6, wherein the blood pressure signal (131) is representative of a blood pressure of the subject (104) while the subject (104) is not affected by the pressurized airflow (102).

8. The computer program product according to any one of claims 6 or 7, wherein the method (400) comprises determining a trend over time between the pressurized airflow (102) provided to the subject (104) and the blood pressure signal (131),
wherein determining the desired property for the pressurized airflow (102) is based on the trend.

9. The computer program product according to any one of claims 6-8, wherein the respiratory information comprises information about at least one of a sleep disordered breathing (SDB) event, obstructive sleep apnea (OSA), central sleep apnea (CSA), obesity hypoventilation syndrome, and COPD,
wherein the method comprises:
determining a second correlation between the setting of the respiratory support device (100) and the respiratory information; and
providing the control signal (140) based on the second correlation.

10. The computer program product according to any one of claims 6-9, wherein the method (400) comprises receiving a medication signal representative of information of a medication use by the subject (104);
determining a third correlation between the setting of the respiratory support device (100) and the medication use;
providing the control signal (140) based on the third correlation.

11. The computer program product according to any one of the preceding claims, the method (400) comprising:
providing an initial control signal to the respiratory support device (100) to provide the pressurized airflow (102) with an initial property before providing the control signal (140);
wherein the respiratory information comprises information about a respiration of the subject (104) while affected by the pressurized airflow (102) with the initial property.

12. The computer program product of claim 11, wherein the method (400) comprises:
providing a plurality of initial control signals to the respiratory support device (100) to provide a plurality of pressurized airflows before providing the control signal (140), wherein each of plurality of pressurized airflows has a different initial property;
for each of the plurality of initial control signals:
receiving a blood pressure signal (131) representative of a blood pressure of the subject (104) while affected by the corresponding pressurized airflow (102);
receiving a respiratory signal (121) representative of respiratory information of the subject (104) while affected by the corresponding pressurized airflow (102);
the method comprises selecting one of the plurality of pressurized airflows as the desired pressurized airflow (102) based on the blood pressure signal (131) and the respiratory signal (121) corresponding to the one of the plurality of initial pressures, and
providing the control signal (140) based on the initial control signal corresponding to the desired pressurized airflow (102).

13. A respiratory support device (100) for providing a pressurized airflow (102) to a subject (104), the respiratory support device (100) comprising:
a processing unit (110) configured to execute the computer program product of any one of claims 1-12;
a sensor interface (113) configured to communicate with at least one sensor (120) to provide the respiratory signal (121) from the at least one sensor (120) to the processing unit (110),
an airflow source (101) adapted to generate the pressurized airflow (102) under control of the control signal (140); and
a patient interface (103) adapted to provide the pressurized airflow (102) to the subject (104).

14. A method (600) for providing a further desired property for a further respiratory support device (100) to provide a further pressurized airflow (102) with the further desired property to a further subject, the method (600) comprising:
receiving (601), from the respiratory support device (100) according to claim 13 that carried out with the processing unit (110) the computer program product according to any one of claims 6-10, a correlation signal (511, 512, 513) representative of at least one of the first correlation, the second correlation and the third correlation;
receiving (602) a further blood pressure signal (514) representative of a blood pressure of the further subject;
determining (603) the further desired property of the further pressurized airflow based on correlation signal (511, 512, 513) and the further blood pressure signal (514).

15. A data-processing system (500), configured to perform the method (600) of claim 14, wherein the data-processing system (500) comprises:
an input interface (501) to receive the correlation signal (511, 512, 513) and the further blood pressure signal (514);
a processing unit (502) configured to determine the further desired property based on the correlation signal (511, 512, 513) and the further blood pressure signal (514); and
an output interface (503) configured to output an output signal (515) representing the further desired property.
